# EUROPEAN PATENT SPECIFICATION

(11) **EP 0 915 156 B1**
(45) Date of publication and mention of the grant of the patent: **31.05.2006**
(21) Application number: 97946124.1
(22) Date of filing: 05.12.1997
(51) Int. Cl.: C12N 15/12, C07K 14/47, C12Q 1/68, A61K 38/17, C12P 21/02, G01N 33/53, G01N 33/577

(54) **IgA NEPHROPATHY-ASSOCIATED GENE**
GEN ASSOZIIERT MIT IgA GLOMERULONEPHRITIS
GENE ASSOCIE A LA GLOMERULONEPHRITE A IgA

(30) Priority: 05.12.1996 JP 32576396
(43) Date of publication of application: 12.05.1999
(73) Proprietor: Nihon University, Tokyo 102-8275 (JP)
(72) Inventor: ISHIWATA, Tetsuyoshi, Machida-shi, Tokyo 194 (JP); SAKURADA, Mikiko, San Diego, CA 92122 (US); NISHIMURA, Ayako, Tokyo 156 (JP); NAKAGAWA, Satoshi, Machida-shi, Tokyo 194 (JP); NISHI, Tatsunari, Tokyo 145 (JP); KUGA, Tetsuro, Machida-shi, Tokyo 194 (JP); SAWADA, Shigemasa, Musashino-shi, Tokyo 180 (JP); TAKEI, Masami, Sayama-shi, Saitama 350-13 (JP)
(74) Representative: Vossius & Partner
(86) International application number: PCT/JP1997/004468
(87) International publication number: WO 1998/024899

(56) References cited:
- DATABASE EMBL [Online] 10 October 1996 (1996-10-10) "Human cosmid LUCA22" retrieved from EBI Database accession no. U73168 XP002211419
- DATABASE SWISSPROT [Online] 1 October 1996 (1996-10-01) "LUCA15" retrieved from EBI Database accession no. P52756 XP002211420
- DATABASE EMBL [Online] 15 August 1996 (1996-08-15) retrieved from EBI Database accession no. N89899 XP002238226
- LAI KAR NENG ET AL: "Increased mRNA encoding for transforming factor-beta in CD4+ cells from patients with IgA nephropathy." KIDNEY INTERNATIONAL, vol. 46, no. 3, 1994, pages 862-868, XP001096449 ISSN: 0085-2538
- PLANT PHYSIOL., Vol. 106, (1994), NEWMAN T. et al., "Genes Galore: A Summary of Methods for Accessing Results from Large-Scale Partial Sequencing of Anonymous Arabidopsis cDNA Clones", p. 1241-1255.
- CLIN. EXP. IMMUNOL., Vol. 103, (1996), H. ICHINOSE et al., "Detection of Cytokine mRNA-Expressing Cells in Peripheral Blood of Patients with IgA Nephropathy Using Non-Radioactive in Situ Hybridization", p. 125-132.
- KIDNY INTERNATIONAL, Vol. 2, (1996), HUNLEY T.E. et al., "Angiotensin Converting Enzyme Gene Polymorphism: Potential Silencer Motif and Impact on Progression in IgA Nephropathy", p. 571-577.
- FEBS LETTERS, Vol. 351, (1994), T. ITO et al., "Fluorescent Differential Display: Arbitrarily Primed RT-PCR Finger-Printing on an Automated DNA Sequencer", p. 231-236.

## Description

### TECHNICAL FIELD

The present invention relates to an isolation of a novel gene and a process for isolating the gene according to a differential display method taking note of an mRNA whose expression level fluctuates in leukocytes of IgA nephropathy patients in comparison with leukocytes of healthy persons. Also, the present invention relates to a novel protein, an antibody recognizing the protein, a DNA encoding the protein, a method for detecting the protein and the DNA, and diagnosis and treatment of IgA nephropathy.

### BACKGROUND ART

IgA nephropathy is a chronic nephropathy which is characterized in that an IgA immune complex considered to be originated from blood deposits in glomerulus of the kidney. In Japan, the IgA nephropathy occupies 30% or more of primary renal diseases, and is the most common renal disease. Moreover, 15 to 30% of the patient with IgA nephropathy achieve renal failure due to poor prognosis. However, since the underlying cause of IgA nephropathy is still unclear, a fundamental therapeutic method has not been found. Additionally, definite diagnosis of IgA nephropathy imposes heavy burden on patients, because the method is carried out by removing a portion of the kidney by biopsy and recognizing deposition of the IgA immune complex in mesangium by means of an immunological staining.

It has been reported that about 50% of the patients with IgA nephropathy have a high blood IgA level *[Diseases of the Kidney,* 5th edition (1993), *Nephron,* 29, 170 (1981)]. It is considered that B cells relate to the production of IgA in blood and T cells relate to the regulation of the production. Furthermore, it has been reported that the production of cytokine, such as interleukin 4, interleukin 5, interleukin 6 or TGF-β (transforming growth factor-β), is high in peripheral T cells of IgA nephropathy patients in comparison with healthy persons [*Clinical & Experimental Immunology*, 103, 125 (1996), *Kidney International,* 46, 862 (1994))] and that integrin, such as VLA (very late activation)-4 and VLA-5, are strongly activated in peripheral lymphocytes of IgA nephropathy patients [*Nephrology, Dialysis, Transplantation,* 10, 1342 (1995)]. On the basis of these facts, it is considered that, in IgA nephropathy, excessive IgA is produced due to abnormality in the immune system, the resulting IgA immune complex in blood deposits on the glomerulus, and the complement system is activated on the deposited IgA immune complex and the like to exert influence and cause disorders of the glomerulus. However, the cause of IgA nephropathy has not yet been determined.

### DISCLOSURE OF THE INVENTION

Elucidation of the cause of IgA nephropathy, as well as a treatment or diagnosis which can reduce a burden on patients are long-sought. The present invention provides a novel DNA related to IgA nephropathy, a method for isolating the DNA, a novel protein related to IgA nephropathy, an antibody recognizing the protein, a DNA encoding the protein, and a therapeutic drug and a diagnostic drug using them.

The present invention relates to a DNA related to IgA nephropathy gene, comprising the nucleotide sequence represented by SEQ ID NO:31. The present invention relates to a method for detecting mRNA of an IgA nephropathy-related gene using the DNA of the present invention and the nucleotide sequence complementary to the DNA; and an IgA nephropathy diagnostic agent comprising the DNA of the invention. The present invention relates to a method for inhibiting transcription of an IgA nephropathy-related gene or translation of the mRNA comprising using the DNA of the present invention and the nucleotide sequence complementary to the DNA; and an IgA nephropathy therapeutic agent comprising said DNA. The present invention relates to a method for isolating an IgA nephropathy gene from leukocytes of a patient with IgA nephropathy comprising conducting a differential display method.

In the present invention, in order to obtain a novel gene, the differential display method [*FEBS Letters,* 351, 231 (1994)] which takes note of the difference in the expression quantity of mRNA in leukocytes between patients with IgA nephropathy and healthy persons is used. The differential display method is a method in which cloning of a novel gene is carried out using pattern of manifestation as an index. That is, an amplified cDNA fragment of a novel gene whose expression level increases or decreases significantly in leukocytes of a patient with IgA nephropathy as compared with leukocytes of a healthy person is obtained by subjecting total RNA or mRNA extracted from cells to the polymerase chain reaction (PCR) using various primers. This method is described below.

Examples of the method for the preparation of a total RNA from leukocytes of patients with IgA nephropathy and leukocytes of healthy persons include guanidine thiocyanate-cesium trifluoroacetate method [*Methods in Enzymol. ,* 154, 3 (1987) 1 , the AGPC method [(*Jikken Igaku,* 9, 1937 (1991)], RNA easy kit for recovering RNA (produced by QIAGEN), and the like.

Examples of the method for preparing poly(A)⁺ RNA from the total RNA include oligo (dT) -immobilizad cellulose column method *(Molecular Cloning, A Laboratory Manual,* 2nd ed.) and the like. Also, examples of the kit for preparing mRNA from leukocytes of patients with IgA nephropathy and leukocytes of healthy persons include Fast Track mRNA Isolation Kit (manufactured by Invitrogen), Quick Prep mRNA Purification Kit (manufactured by Pharmacia), and the like .

Using an anchor primer, cDNA is synthesized in the usual way from the RNA extracted by the above-described method from leukocytes of a patient with IgA nephropathy or leukocytes of a healthy person, and PCR is carried out using an anchor primer having a 5'-end labeled with fluorescence and an arbitrary primer. The anchor primer is a primer in which an oligonucleotide of adenine, guanine or cytosine, excluding thymidine, is added to the 3'-end of an oligo(dT) sequence which hybridizes with a 3' -end poly (A) sequence of mRNA. The arbitrary primer is an oligonucleotide which amplifies various cDNA sequences and can yield a large number of amplified cDNA fragments by a single reaction. Preferably, the oligonucleotide has a length of about 10 mer.

After the PCR, each of the amplified cDNA is subjected to polyacrylamide gel electrophoresis, and the fluorescence is detected with a fluorimager. By comparing the electrophoresis patterns of the amplified cDNA fragments derived from leucocytes of a patient with IgA nephropathy, the cDNA fragment in which the expression amplification is fluctuated is cut from the gel, the amplified cDNA fragment is inserted into a vector, and the nucleotide sequence of the DNA is determined by a usually used nucleotide sequence analyzing method such as the dideoxy method of Sanger *et al*. [*Proc*. *Natl. Acad. Sci. USA*, 74, 5463 (1977)], or the like.

Examples of the vector to which the DNA fragment is inserted include pDIRECT [*Nucleic Acids Research*, 18, 6069 (1990)], pPCR-Script Amp [manufactured by Stratagene, Strategies, 5, 6264 (1992)], pT7Blue (manufactured by Novagen), pCR II [manufactured by Invitrogen, *Biotechnology*, 9, 657 (1991)], pCR-TRAP (manufactured by Genehunter) pNoTA_{T7} (manufactured by 5'→3') and the like.

The analysis of the nucleotide sequence is carried out by using a nucleotide sequence automatic analyzer, such as 373A•DNA sequencer (manufactured by Applied Biosystems) and the like.

The DNA of the present invention includes a DNA comprising the nucleotide sequence represented by SEQ ID NO:31.

Examples of a method for detecting the mRNA related to IgA nephropathy using the DNA of the present invention include Northern hybridization [*Molecular Cloning, A Laboratory Manual*, 2nd ed., Cold Spring Harbor Laboratory Press (1989)], PCR [*PCR Protocols,* Academic Press. (1990)], and the like. Particularly, RT (reverse transcribed)-PCR is simple and easy and can therefore be applied to the diagnosis of IgA nephropathy. Specifically, the amplified fragment is detected by collecting blood from human to recover leucocyte, transforming the RNA isolated therefrom into cDNA using an oilgo (dT) primer and a reverse transcriptase into, and conducting PCR using a pair of oligonucleotide primers corresponding to the mRNA to be detected.

Examples of the oligonucleotide primers include a sense primer corresponding to the 5'-end side nucleotide sequence, and an antisense primer corresponding to the 3'-end side nucleotide sequence, of a portion of the mRNA to be detected. In this case, the base corresponding to uracil in mRNA corresponds to thymidine in the oligonucleotide primer.

As the sense primer and antisense primer, it is preferred to use oligonueleotides in which melting point (Tₘ) and the number of bases are not significantly different from each other. Preferably, the base number is 15 to 40 mer.

The nucleotide sequence moiety to be amplified using the above oligonucleotide primer may be any nucleotide sequence region of the mRNA, but a nucleotide sequence region which has a length of 50 bp to 2 kbp and does not contain a sequence rich in a repeating sequence or GC (guanine-cytosine) bases is preferred.

Furthermore, similarly, the IgA nephropathy can be treated by inhibiting the transcription of DNA or translation of mRNA using an antisense RNA/DNA *[Chemistry,* 46, 681 (1991), *Biotechnology,* 9, 358 (1992)].

The inhibition of production of the protein using anti-sense RNA/DNA technology can be carried out by designing and preparing an oligonucleotide based on the nucleotide sequence of a portion of the DNA encoding the protein of the present invention, preferably that of 10 to 50 bases positioned in the translation initiation domain, and administrating it *in vivo.* As the nucleotide sequence of the synthesis oligonucleotide, those which partially conforms the nucleotide sequence of the anti-sense chain of the DNA encoding the protein of the present invention, or those which have been modified to the extent not to lose the activity of inhibiting the expression of the protein activity can be used. As oligonucleotide, DNA, RNA or their derivatives, such as methyl or derivatives, can be used.

In order to obtain a full-length DNA from cDNA fragments obtained by the above-described method, screening from various cDNA libraries can be carried out by means of hybridization using the above-described amplified cDNA fragments as a probe. The method for preparing a cDNR library is described below.

Examples of the method for the preparation of the cDNA library include methods described in *Molecular Cloning, A Laboratory Manuel,* 2nd. ed., or *Current Protocols in Molecular Biology, Supplement 1 to 34*, methods using a commercially available kit, such as SUPERSCRIPT^{™} Plasmid system for cDNA Synthesis and Plasmid Cloning (manufactured by Life Technologies) or ZAP-cDNA Synthesis Kit (manufactured by Stratagene), and the like. Additionally, several cDNA libraries are commercially available, including a cDNA library which can be used in the present invention such as a human leukocyte cDNA library manufactured by Life Technologies and the like.

In the preparation of the cDNA library, the vector to which the cDNA, synthesized using mRNA extracted from cell as a template, is inserted may be any vector so long as the cDNA can be inserted thereto. Examples include ZAP express [*Strategies*, 5, 58 (1992)], pBluescript II SK(+) [*Nucleic Acids Research*, 17, 9494 (1989)], λ ZAP II (manufactured by Stratagene), λgt10, λgt11 [*DNA Cloning, A Practical Approach,* 1 , 49 (1985)], λExCell (manufactured by Pharmacia), pcD2 [*Mol*. *Cell. Biol.,* 3, 280 (1983)], pUC18 [*Gene*, 33, 103 (1985)], and the like. With regard to the *Escherichia coli* for introducing the cDNA library constituted by the vector, any microorganism belonging to *Escherichia coli* can be used so long as the introduction, expression and maintenance of the cDNA library can be conducted. Examples include *Escherichia coli* XL1-Blue MRF' [*Strategies*, 5, 81 (1992)], *Escherichia coli* C600 [*Genetics*, 39, 440 (1954)], *Escherichia coli* Y1088, *Escherichia coli* Y1090 [*Science,* 222, 778 (1983)], *Escherichia coli* NM522 [*J*. *Mol. Biol.,* 166, 1 (1983)], *Escherichia coli* K802 [*J*. *Mol. Biol.*, 16, 118 (1966)], *Escherichia coli* JM105 [*Gene,* 3B, 275 (1985) 1, and the like.

The cDNA can be also obtained without preparing a cDNA library by the 5,'-RACE (rapid amplification of cDNA ends) and 3'-RACE [*Proc*. *Natl. Acad. Sci. USA,* 85, 8998 (1988)] in which adapters are added to both ends of the cDNA and then PCR is carried out using primers based on the nucleotide sequence of the adapter and the nucleotide sequence of the amplified fragment. Alternatively, the cDNA can be obtained by PCR based on the nucleotide sequence or a chemical synthesis method using a DNA synthesizer. A cDNA clone can be selected from the cDNA library according to a colony hybridization or plaque hybridization method (*Molecular Cloning, A Laboratory Manual,* 2nd ed.) using a probe labeled with an isotope or fluorescence. The cDNA may be also prepared according to the polymerase chain reaction (PCR) (*Molecular Cloning, A Laboratory Manual,* 2nd ed. or *Current Protocols in Molecular Biology, Supplement 1 to 34)* by preparing a primer and using, as a template, cDNA synthesized from poly (A)⁺RNA or mRNA, or cDNA library.

The nucleotide sequence of the DNA can be determined by cleaving the cDNA clone selected by the above method with an appropriate restriction enzyme, cloning to a plasmid, such as pBluescript KS(+) (manufactured by Stratagene) or the like, and then analyzing by a conventional nucleotide sequence analysis method, such as dideoxy method of Sanger *et al.* [*Proc. Natl. Acad. Sci., U.S.A.,* 74, 5463 (1977)] or the like. The nucleotide sequence can be analyzed by using a nucleotide sequence automatic analyzer, such as 373A•DNA sequencer (manufactured by Applied Biosystems) or the like.

Confirmation of novelty of the thus obtained nucleotide sequence is carried out using nucleotide sequence data bases, such as GenBank, EMBL, DDBJ, and the like.

The examples of the present invention are shown below.

### [EXAMPLES]

### Example 1 Differential display of leukocytes of IgA nephropathy patients and healthy persons

### (1) Preparation, of total RNA from leukocytes of IgA nephropathy patients and healthy persons

A 20 ml portion of blood was collected from each of five IgA nephropathy patients and five healthy persons. This was mixed with 500 µl of 1,000 units/ml heparin sodium solution (manufactured by Shimizu Seiyaku) to inhibit coagulation, transferred into a centrifugation tube and then contrifuged at 3,300 rpm for 15 minutes at room temperature, and the resulting intermediate layer buffy coat containing leukocytes was transferred into another centrifugation tube. Thereafter, total RNAs were obtained in accordance with the AGPC method [*Experimental Medicine,* 9, 1937 (1991)] or using an RNA recovering kit RNA easy (manufactured by QIAGEN).

### (2) Fluorescence differential display using leukocyte total RNAs of IgA nephropathy patients and healthy persons

Distilled water was added to 2.5 µg of each of the total RNAs to a total volume of 9 µl, and the solution was mixed with 1 µl of an anchor primer (50 µM, custom-synthesized by Sawady Technology) whose 5' -end had been fluorescence-labeled with fluorescein isothiocyanate (referred to as "FITC" hereinafter), heated at 70°C for 5 minutes and then immediately cooled on an ice bath. Since each of the three primers FAH (FAH: 5' -FITC-GT₁₅A-3'), FGH (FGH: 5'-FITC-GT₁₅G-3') and FCH (FCH: 5'-FITC-GT₁₅C-3') was used in each reaction as the fluorescence-labeled anchor primer, a total of three combinations of reactions were carried out for one sample of total RNAs. A 4 µl portion of 5 × reverse transcriptase reaction buffer [250 mM tris(hydroxymethyl)aminomethane (Tris)-HCl (pH 8.3), 375 mM KCl, 15 mM M4Cl₂] was mixed with 2 µl of 100 mM dithiothreitol (DTT), 1 µl of 10 mM dNTP (dATP, dGTP, dTTP and dCTP), 1 µl of distilled water and 1 µl (200 units) of a reverse transcriptase SUPERSCRIPT II RNase. H⁻ Reverse Transcriptase (manufactured by Life Technologies), and the resulting mixture was allowed to stand at room temperature for 10 minutes, allowed to react at 42°C for 50 minutes to synthesize a cDNA, and then heated at 90°C. for 5 minutes to terminate the reaction. To the reaction solution was added 40 µl of TE buffer [10 mM Tris-HCl (pH 8.0), 1 mM disodium ethylenediaminetetraacetate (EDTA) (pH 8.0)].

Subsequently, next, 14.7 µl of distilled water, 2 µl of 10 × PCR buffer [100 mM Tris-HCl (pH 8.8), 500 mM KCl, 15 mM MgCl₂, 1% Triton X-100], 0.8 µl of 2.5 mM dNTP, 0.3 µl of 50 µM fluorescence-labeled anchor primer (the same among FAB, FGH. and FCH used in the cDNA synthesis), 1 µl of 10 µM. arbitrary primer (manufactured by Operon Technologies) and 0.2 µl of DNA polymerase Gene Taq. (5 units/µl, manufactured by Nippon Gene) were added to 1. µl of each of the thus synthesized cDNA samples, and the resulting mixture was arranged in Thermal Cycler. The PCR was effected by carrying out the reaction at 94°C for 3 minutes, 40°C for 5 minutes and 72°C for 5 minutes, subsequently carrying out a total of 27 cycles of the reaction in which one cycle was comprised of the steps of 95°C for 15 seconds, 40°C for 2 minutes and 72°C for 1 minute, and finally carrying out 5 minutes of the reaction at 72°C. Since each reaction was carried out by a combination of one of the above-described three types as the fluorescence-labeled anchor primer with one of 60 types of OPD-1 to 20, OPE-1 to 20 and OPV-1 to 20 manufactured by Operon Technologies as the arbitrary primer, a total of 180 reactions, and since a reaction of the fluorescence-labeled anchor primer FGH with an arbitrary primer OPB-2 (manufactured by Operon Technologies) was also carried out, a total of 181 reactions were carried out for the total RNAs.

A 4 µl portion of each of the PCR reaction solutions was mixed with 3 µl of electrophoresis sample buffer use (95% formamide, 0.1% xylene cyanol, 0.1% Bromophenol Blue), and the mixture was heated at 95°C for 2 minutes, immediately cooled thereafter on an ice bath and then subjected to 2.5 hours of 6% acrylamide gel electrophoresis at 1, 500 V. A solution composed of 89 mM Tris, 89 mM boric acid and 2 mM EDTA was used as the electrophoresis buffer. By measuring fluorescence of the gel after electrophoresis using Fluorimager (manufactured by Molecular Dynamics), the fragments amplified by PCR were detected and compared. In comparison with 5 cases of the healthy persons, a band which significantly increased or decreased in leukocytes of 5 cases of the IgA nephropathy patients was recorded.

Total RNAs were prepared from other 3 cases of IgA other nephropathy patients and 3 cases of other healthy persons in the same manner to carry out the differential display in the same manner. A total of 197 bands which showed increased or decreased fluorescence in both of the above two trials of the differential display were cut off from the gels.

A 38 µl portion of distilled watery 5. µl of 10 x PCR buffer, 4 µl of 2.5 mM dNTP, 0.6 µl of an anchor primer (no fluorescence labeling: 34 µM, custom-synthesized by Sawady Technology), 2 µl of 10 µM arbitrary primer and 0.5 µl of. DNA polymerase Gene Taq were added to about 1/4 portion of each of the gels thus cut off, the resulting mixture was heated at 94°C for 3 minutes and then a total of 30 cycles of the reaction was carried out in which one cycle was comprised of the steps of 95°C for 15 seconds, 40°C for 2 minutes and 72°C for 1 minute, subsequently carrying out 5 minutes of the reaction at 72°C to complete PCR. The same combinations of anchor primers with optional primers used in the first differential display method were employed. Each of the resulting reaction, solutions was extracted with phenol-chloroform (1:1) and then with chloroform-isoamyl alcohol (24:1), subsequently carrying out ethanol precipitation. To purify the precipitate, 1.5% low melting point agarose gel (SEA PLAQUE GTG, manufactured by FMC Bioproducts) electrophoresis was carried out. After the electrophoresis, the resulting gels were stained with ethidium bromide and then the bands containing amplified fragments were cut off. The gel was heated at 65°C for 15 minutes to melt agarose and then extracted with phenol-chloroform. After chloroform-isoamyl alcohol extraction, the thus obtained extract was subjected to ethanol precipitation and the resulting precipitate was dissolved in 10 µl of TE buffer.

A 1 µl portion of each of the amplified fragments was mixed with 1 µl of a vector for PCR fragment cloning use, pT7BlueT-Vector (manufactured by Novagen), and the amplified fragment was cloned into the plasmid using DNA Ligation Kit ver.1 (manufactured by Takara Shuzo) in accordance with the manual attached to the kit. *Escherichia coli* DH5α (manufactured by Gibco BRL) was transformed in accordance with a known method, and the ampicillin-resistant transformant was obtained. The transformant colony was suspended in 20 µl of distilled water, the suspension was mixed with 2.5 µl of 10 × PCR buffer, 2 µl of 2.5 mM- dNTP, 0.3 µl of 34 µM anchor primer, 1 µl of 10 µM arbitrary primer and 0.5 µl of a DNA polymerase Gene Taq, and the mixture was subjected to PCR under the same conditions of the above-described re-amplification of amplified fragments and then analyzed by electrophoresis which recognized that an amplified fragment has the same length as in the first differential display and therefore, the amplified fragment was cloned into the plasmid.

Nucleotide sequence of the amplified fragment was determined using DNA Sequencer (manufactured by Perkin Elmer). In carrying out the nucleotide sequence determination, Dye Primer Cycle Sequencing Kit manufactured by Perkin Elmer and the method described in the manual attached to the kit were used. Using restriction enzymes capable of cleaving restriction enzyme sites in the determined nucleotide sequence, the reaction product obtained by the above-described differential display was cleaved and then subjected to electrophoresis to recognize that the position of electrophoresis band corresponding to the thus cut off amplified fragment was changed. Each of the thus obtained nucleotide sequences was compared with a nucleotide sequence data base GenBank to select a total of 66 clones which were not present among the known nucleotide sequences in the data base or coincided only with the expressed sequence tag among nucleotide sequences in the data base.

### Example 2 Detection of specificity of mRNA expression by RT-PCR

Using 2 µg of each of the total RNA samples obtained in Example 1 from leukocytes of five cases of IgA nephropathy patients and 5 cases of healthy persons single-stranded cDNA was synthesized using a single-stranded cDNA synthesis kit Superscript Preamplification System (manufactured by Life Technologies) by the oligo (dT) primer attached to the kit. Specific reagents and method employed were as described in the protocol attached to the kit. A 21 µl portion of solution after the reaction was adjusted to a total volume of 420 µl by adding 399 µl of distilled water, and a 10 µl portion of the thus prepared solution was used in the detection of the expression quantity of mRNA corresponding to each amplified fragment by RT-PCR. That is, 10 µl of the leukocyte single-stranded cDNA solution was mixed with 15.8 µl of distilled water, 4 µl of 10 × PCR buffer, 3.2 µl of 2.5 mM dNTP, 2 µl of DMSO, 2 µl of 10 µM gene-specific sense primer, 2 µl of 10 µM gene-specific antisense primer and 2. µl of a DNA polymerase Gene Taq which had been diluted to 1 unit/µl, and the resulting mixture was heated at 94°C for 5 minutes, cooled on an ice bath for 5 minutes and then subjected to a total of 24 to 35 cycles of PCR in which one cycle was comprised of the steps of 95°C for 30 seconds, 65°C for 1 minute and 72°C for 2 minutes. After carrying out 2% agarose gel electrophoresis, the gel was stained with 0.01% Cyber Green (manufactured by Takara Shuzo), and the amount of the amplified fragment determined by Fluorimager was used as relative expression quantity of mRNA.

In order to make a correction of the amount of mRNA, the same reaction was carried out on a housekeeping gene, glyceraldehyde 3-phosphate dehydrogenase (G3PDH) gene, using specific primers (5'-CCCATCACCATCTTCCAGGAGC-3',
5'-TTCACCACCTTCTTGATGTCATCATA-3') and the expression level of mRNA for each gene was corrected based on the ratio of the expression level of G3PDH mRNA, and then the average value of five cases of IgA nephropathy patients and the average value of 5 cases of healthy persons were compared and 31 gene clones having a difference in their values were selected as genes whose expression quantity was changed in patients with IgA nephropathy. The thus selected genes are summarized in Tables 1-1 and 1-2.

**Table 1-1**

| SEQ ID NO | Gene | Amplification primer¹⁾ | bp²⁾ | Expression fluctuation³⁾ | RT-PCR primer⁴⁾ | RT-PCR cycle number |
|---|---|---|---|---|---|---|
| 1 | IMP377-A | FGH/OPD-1 | 256 | 5.0 | 55, 56 | 28 |
| 2 | INM0 63-7 | FGH/OPB-2 | 155 | 12.5 | 33, 34 | 28 |
| 3 | INP303-A | FRH/OPD-5 | 305 | 9.9 | 35, 36 | 28 |
| 4 | INM315-10 | FAH/OPD-9 | 278 | 2.8 | 37, 38 | 35 |
| 5 | INP319-3 | FAA/OPD-10 | 135 | 14.4 | 39, 40 | 28 |
| 6 | INP324-A | FAH/OPD-12 | 197 | 19.9 | 41, 42 | 28 |
| 7 | INP332-A | FAH/OPD-16 | 137 | 16.6 | 43, 44 | 28 |
| 8 | INM335-3 | FAH/OPD-17 | 274 | 4.2 | 45, 46 | 28 |
| 9 | INM336-A | FAH/OPD-17 | 171 | 0.14 | 47, 48 | 28 |
| 10 | INM351-10 | FCH/OPD-4 | 161 | 1.8 | 49, 50 | 28 |
| 11 | INP356-4. | FCH/OPD-7 | 323 | 18.5 | 51, 52 | 35 |
| 12 | INP364-A | FCH/OPD-12 | 138 | 3.8 | 53, 54 | 28 |
| 13 | INP379-A | FGH/OPD-2 | 244 | 8.6 | 57, 58 | 35 |
| 14 | INP380-A | FGH/OPD-2 | 135 | 1-5.7 | 59, 60 | 35 |
| 15 | INP401-A | FGH/OPD-20 | 258 | 16.7 | 61, 62 | 24 |
| 16 | INM403-A | FAH/OPE-3 | 219 | 2.3 | 63, 64 | 28 |
| 17 | INP407-A | FAH/OPE-5 | 191 | 9.1 | 65, 66 | 28 |
| 18 | INM408-A | FAH/OPE-5 | 148 | 0.65 | 67, 68 | 28 |
| 19 | INP410-5 | FAH/OPE-6 | 306 | 2.0 | 69, 70 | 28 |
| 20 | INM419-14 | FAH/OPE-11 | 357 | 0.064 | 71, 72 | 35 |

**Table 1-2**

| | | | | | | |
|---|---|---|---|---|---|---|
| 21 | IMP429-A | FGH/OPE-7 | 219 | 2.4 | 73, 74 | 28 |
| 22 | INP431-A | FGH/OPE-8 | 251 | 13.1 | 75, 76 | 24 |
| 23 | INP43e-A | FGH/OPE-11 | 233 | 5.4 | 77, 78 | 24 |
| 24 | INP444-A | FGH/OPE-15 | 176 | 3.3 | 79, 80 | 24 |
| 25 | INP451-2 | FCH/OPE-4 | 241 | 14.0 | 81, 82 | 32 |
| 26 | INP458-A | FCH/OPE-11 | 217 | 9.2 | 83, 84 | 28 |
| 27 | INP463-A | FCH/OPE-19 | 232 | 18.2 | 85, 86 | 35 |
| 28 | INP470-A | FCH/OFV-4 | 228 | 5.8 | 87, 89 | 28 |
| 29 | INP482-A | FCH/OPV-10 | 298 | 9.9 | 89, 90 | 28 |
| 30 | INP485-6 | FCH/OPV-17 | 291 | 8.5 | 91, 92 | 28 |
| 31 | GTINP332A-⁵⁾ | - | 869 | 4.6 | 93, 94 | 24 |

1) : A combination of the anchor primer with the arbitrary primer used in the differential display is shown.
2): The length of the amplified fragment of the differential display is shown, excluding GTINP332A-21.
3): Expression fluctuation is shown as the value of "the average value of mRNA expression levels in 5 cases of IgA nephropathy patients/the average value of mRNA expression levels in 5 cases of healthy persons".
4) : The primer used in the RT-PCR is shown by the SEQ ID NO.
5) : GTINP332A-21 is not a gene from which its amplified fragment was obtained by the differential display, but is a cDNA clone obtained from a transformant when an attempt was made to obtain a cDNA clone of full-length length INP332-A from a human leukocyte cDNA library in the same manner as described in Example 3. This gene was included in this table, because, when a portion of its cDNA nucleotide sequence was determined, this was found to be a cDNA clone of a novel gene whose nucleotide sequence is different from that of INP332A, and the result of PCR carried out in Example 2 based on its nucleotide sequence showed that expression of mRNA was increased in leukocytes of IgA nephropathy patients in comparison with the case of healthy persons.

Thus, it becomes possible to carry out diagnosis of IgA nephropathy by observing the expression levels of these genes in the leukocytes samples to be tested by PT-PCR using primers of these genes and mRNAs of the samples.

### Example 3 Cloning of whole length cDNA

### (1) Isolation of INP377-A cDNA clone

A INP377-A cDNA clone was obtained from a human leukocyte cDNA library (manufactured by Life Technologies) in which pCMV-SPORT (manufactured by Life Technologies) was used as the vector, using GENE TRAPPER cDNA Positive Selection System (manufactured by (Life Technologies). That is, the clone of interest was isolated by making clones in the cDNA library into single-stranded chains using Gene II protein and exonuclease III, carrying out their hybridization with a probe, namely a biotinated complimentary oligonueleptide (the sense primer used in Example 2 was used) which corresponds to the INP377-A gene, and then allowing the probe to bind to magnetic beads to which streptoavidin has been added. The thus hybridized single-stranded cDNA was released from the probe, made into double-stranded chain using a DNA polymerase and then transformed into *Escherichia coli*, thereby obtaining the INP377-A cDNA clone as an ampicillin resistant strain. Specific reagents and method employed were as described in the protocol attached to the kit. Each of the transformant colonies was suspended in 18 µl of distilled water, the suspension was mixed with 2.5 µl of 10 × PCR buffer, 2 µl of 2.5 mM dNTP, 1 µl of 10 µM gene-specific sense primer, 1 µl of 10 µM gene-specific antisense primer and 0.5 µl of a DNA polymerase Gene Taq, and the resulting mixture was subjected to PCR under the same conditions of RT-PCR, subsequently carrying out an electrophoresis to isolate a transformant as the INP377-A cDNA clone of interest in which an INP377-A cDNA fragment of about 200 bp deduced from the positions of primers was amplified.

Plasmid DNA was isolated from this clone in accordance with the known method (*Molecular Cloning: A laboratory manual*, 2nd ed.), and the plasmid was named pGTINP377A-46C. In addition, the plasmid DNA was digested with restriction enzymes *Sal*I*.* and *Not*I (both manufactured by Takara Sbuzo) and then subjected to agarose gel electrophoresis to find that the cDNA, has a size of about 3 kb.

### (2) Determination of INP377-A cDNA nucleotide sequence

Nucleotide sequence of INP377-A cDNA in pGTINP377A-46C was determined using 377 DNA Sequencer manufactured by Perkin-Elmer. With regard to specific reagents and method used in the nucleotide sequence determination, Dye Primer Cycle Sequencing FS Ready Reaction Kit manufactured by Perkin-Elmer was used in accordance with the instructions of the kit. The thus determined nucleotide sequence is shown in SEQ ID NO:1. An open reading frame (CRT) corresponding to 143 amino acids was present in this nucleotide sequence. When the 377-A cDNA nucleotide sequence was compared with a data base, it was found that its partial sequence corresponding to N-terminal 137 amino acids coincides with the partial sequence of the human gene LUCA15, which corresponds to N-terminal 137 amino acids having homology with a Drosophila cancer inhibition gene Sx1, but a nucleotide sequence having no homology continues thereafter, and the sequence obtained by the differential display is present in this nucleotide sequence having no homology.

### INDUSTRIAL APPLICABILITY

IgA nephropathy can be diagnosed and treated by using the novel gene obtained according to the present invention.

### SEQUENCE LISTING

SEQ ID NO:1
   SEQUENCE LENGTH: 2689
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:1
   SEQUENCE LENGTH: 2660
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:2
   SEQUENCE LENGTH: 155
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:3
   SEQUENCE LENGTH: 305
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:4
   SEQUENCE LENGTH: 278
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:5
   SEQUENCE LENGTH: 135
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:6
   SEQUENCE LENGTH: 197
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:7
   SEQUENCE LENGTH: 137
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:8
   SEQUENCE LENGTH: 274
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:9
   SEQUENCE LENGTH: 171
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:10
   SEQUENCE LENGTH: 161
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO: 11
   SEQUENCE LENGTH: 323
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:12
   SEQUENCE LENGTH: 138
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:13
   SEQUENCE LENGTH: 244
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO: 14
   SEQUENCE LENGTH: 135
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:15
   SEQUENCE LENGTH: 258
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO: 16
   SEQUENCE LENGTH: 219
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO: 17
   SEQUENCE LENGTH: 191
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:18
   SEQUENCE LENGTH: 148
   SEQUENCE TYPE: nucleic acid
   STRANEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:19
   SEQUENCE LENGTH: 306
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:20
   SEQUENCE LENGTH: 357
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:21
   SEQUENCE LENGTH: 219
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:22
   SEQUENCE LENGTH: 251
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:23
   SEQUENCE LENGTH: 233
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:24
   SEQUENCE LENGTH: 176
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:25
   SEQUENCE LENGTH: 241
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:26
   SEQUENCE LENGTH: 217
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:27
   SEQUENCE LENGTH: 233
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:28
   SEQUENCE LENGTH: 228
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:29
   SEQUENCE LENGTH: 298
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:30
   SEQUENCE LENGTH: 291
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:31
   SEQUENCE LENGTH: 869
   SEQUENCE TYPE: nucleic acid
   STRANDEDNESS: double
   TOPOLOGY: linear
   MOLECULE TYPE: cDNA
   ORIGINAL SOURCE
   ORGANISM: human
   CELL TYPE: leukocyte
   SEQUENCE:
SEQ ID NO:32
   SEQUENCE LENGTH: 143
   SEQUENCE TYPE: amino acid
   TOPOLOGY: linear
   MOLECULAR TYPE: protein
   ORIGINAL SOURCE
   ORGANISM: human
   CELL TYPE: leukocyte
   SEQUENCE:
SEQ ID NO:33
   SEQUENCE LENGTH: 26
   SEQUENCE TYPE: nucleic acid
   STRANDEDNESS: single
   TOPOLOGY: linear
   MOLECULE TYPE: other nucleic acid, synthetic DNA
   SEQUENCE:
   GGGCTTAATA TTATTCATAG ATCGAG
SEQ ID NO:34
   SEQUENCE LENGTH: 26
   SEQUENCE TYPE: nucleic acid
   STRANDEDNESS: single
   TOPOLOGY: linear
   MOLECULE TYPE: other nucleic acid, synthetic DNA
   SEQUENCE:
   GTTATTATAC TATCAAGTAA CCCAAC
SEQ ID NO:35
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GTGGATCTGG ATTTTTGTCA TATGT
SEQ ID NO:36
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GTTTGTGATT ATAACCCAAC ATGTG
SEQ ID NO:37
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GAAGGGGAAG AGACATTAAA TTATC
SEQ ID NO:38
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GCTTCTAAAT CTCCTGAGTC ACTT
SEQ ID NO:39
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GACAATGAGT AAGAAGAAAG AGGG
SEQ ID NO:40
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GTCCAGTCCC TTGGTTTATT TGTC
SEQ ID NO:41
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GGTACCCAGT TTCAAATTAA CATGG
SEQ ID NO:42
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GATTCTTCAA CTGCCAAACT TGTTC
SEQ ID NO:43
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GCTGATGCTT TTCTATCTGA CTTC
SEQ ID NO:44
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GACCAGGACT GAACAGAGGT GA
SEQ ID NO:45
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GCTTATAGAC CATGTTTGTA GTAGG
OSEQ ID NO:46
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GTGAACAAAT GCTAAATCAG ACATG
SEQ ID NO:47
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GCCACGGGTT TCCCATATCG AA
SEQ ID NO:48
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GACTATACTT AGGAACCTCT GCAA
SEQ ID NO:49
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GTTCTGCTCT CAGCAGATTG GTTA
SEQ ID NO:50
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GCCKACATCT GAACTAAATA CTGC
SEQ ID NO:51
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GTTCAGTGAA TGTTACCTAG AAACA
SEQ ID NO:52
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GGAGTGAAAA CTGTCTTGTT CATC
SEQ ID NO:53
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GTATGACAAA TAGTTTCTGC CTGAT
SEQ ID NO:54
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GATTAACAAA GATGTACAGA CTGAG
SEQ ID N0:55
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GAGACAGCAT TCAGATATAG ACGG
SEQ ID NO:56
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GCGTGGAATC AAATGGAGTG GC
SEQ ID NO:57
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GATGGCCTGT GTGAACAGAT TAAT
SEQ ID NO:58
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GAGAGAGATG TCAGAGTCAT TAGC
SEQ ID NO:59
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GATCCCCACA ATTTCTTGTG ATTG
SEQ ID NO:60
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GTTCCCCTAA AATAATGTGG TAATG
SEQ ID NO:61
   SEQUENCE LENGTH: 23
   SEQUENCE TYPE: nucleic acid
   STRANDEDNESS: single
   TOPOLOGY: linear
   MOLECULE TYPE: other nucleic acid, synthetic DNA
   SEQUENCE:
   GAGGATACTC TCCAATGGTG ATG
SEQ ID NO:62
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GTCTTAACAT CTAGCCTACT GGAG
SEQ ID NO:63
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GAGAGGAGCC ATGTATACAA ACCA
SEQ ID NO:64
   SEQUENCE LENGTH: 26
   SEQUENCE TYPE: nucleic acid
   STRANDEDNESS: single
   TOPOLOGY: linear
   MOLECULE TYPE: other nucleic acid, synthetic DNA
   SEQUENCE:
   GCACGCAGGA TCAGATATAG TAATTC
SEQ ID NO:65
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GCTGAAACCT AAGCTGAAGGAAGG
SEQ ID NO:66
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GTCCCTCACC TCAGATCACA CC
SEQ ID NO:67
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GCTATCTACC TGGCAGGAAA AGAG
SEQ ID NO:68
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GAGTTTCTTA CTATGATCTG GATTC
SEQ ID NO:69
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GCAAAATGTA CTCAGCTTCA ATCAC
SEQ ID NO:70
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GTAAATGCAG TACTGTTCTG ATCC
SEQ ID NO:71
   SEQUENCE LENGTH: 26
   SEQUENCE TYPE: nucleic acid
   STRANDEDNESS: single
   TOPOLOGY: linear
   MOLECULE TYPE: other nucleic acid, synthetic DNA
   SEQUENCE:
   GAATGCTTCA TTCTCATTGT TTAAGG
SEQ ID NO:72
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GTCACTAGGA TTCCACAGAA CTTC
SEQ ID NO:73
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GAGGTAGGGC TTCCCTTCGC TA
SEQ ID NO:74
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GCATAACAAG TGACAGGGTT AGTTA
SEQ ID NO:75
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GGTGCTCCTT CCTTACACTG GT
SEQ ID NO:76
   SEQUENCE LENGTH: 23
   SEQUENCE TYPE: nucleic acid
   STRANDEDNESS: single
   TOPOLOGY: linear
   MOLECULE TYPE: other nucleic acid, synthetic DNA
   SEQUENCE:
   GACTACACAT AAACCCACCC CAG
SEQ ID N0:77
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GGGTACAGGA TTTCTAAGAA GTGG
SEQ ID NO:78
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GGAGAAAATT TCAGCTCATC TGAAG
SEQ ID NO:79
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GCTGAAGTTA AGCATTAATA CGCC
SEQ ID NO:80
   SEQUENCE LENGTH: 23
   SEQUENCE TYPE: nucleic acid
   STRANDEDNESS: single
   TOPOLOGY: linear
   MOLECULE TYPE: other nucleic acid, synthetic DNA
   SEQUENCE:
   GCGGCTGTAA TGTGCAATGA TGT
SEQ ID NO:81
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GACAGCAACC TAATAACAGC TGTC
SEQ ID NO:82
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GTCCTAGGCA CTTGTCACTA GG
SEQ ID NO:83
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GAGGGGACTT CCAAGAGTCT CT
SEQ ID NO:84
   SEQUENCE LENGTH: 25
   SEQUENCE TYPE: nucleic acid
   STRANDEDNESS: single
   TOPOLOGY: linear
   MOLECULE TYPE: other nucleic acid, synthetic DNA
   SEQUENCE:
   GTCTTCAGGA AAATTGTAGT TACAG
SEQ ID NO:85
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GTTACAAACA CACACGAAGT TCCT
SEQ ID NO:86
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GACTTCCTAA GGCACACTCA GC
SEQ ID NO:87
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GTTTAACTAC CTCTCAGGTC ATGA
SEQ ID NO:88
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GTCGCCAAGG CTGTAGTGCA AT
SEQ ID NO:89
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GAAATAGGTA TCCCTTGATG TCGA
SEQ ID NO:90
   SEQUENCE LENGTH: 24
   SEQUENCE TYPE: nucleic acid
   STRANDEDNESS: single
   TOPOLOGY: linear
   MOLECULE TYPE: other nucleic acid, synthetic DNA
   SEQUENCE:
   GACCAAGAAT TCAGTTCATC AGTT
SEQ ID NO:91
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GAATGAACCA GAGCCAGGAC AG
SEQ ID NO:92
   SEQUENCE LENGTH: 22
   SEQUENCE TYPE: nucleic acid
   STRANDEDNESS: single
   TOPOLOGY: linear
   MOLECULE TYPE: other nucleic acid, synthetic DNA
   SEQUENCE:
   GCCTTGTATG TATGCCTGTG CC
SEQ ID NO:93
   SEQUENCE LENGTH: 21
   SEQUENCE TYPE: nucleic acid
   STRANDEDNESS: single
   TOPOLOGY: linear
   MOLECULE TYPE: other nucleic acid, synthetic DNA
   SEQUENCE:
   AAGAGTCCAC CAGGCCATGG A
SEQ ID NO:94
   SEQUENCE LENGTH: 23
   SEQUENCE TYPE: nucleic acid
   STRANDEDNESS: single
   TOPOLOGY: linear
   MOLECULE TYPE: other nucleic acid, synthetic DNA
   SEQUENCE:
   TACCTTGTGT ACTTCTAGCT GAG

## Claims

1. A DNA related to IgA nephropathy gene, comprising the nucleotide sequence represented by SEQ ID NO: 31.

2. A recombinant DNA comprising the DNA according to claim 1 and a vector.

3. An IgA nephropathy diagnostic agent, comprising the DNA according to claim 1.

4. An IgA nephropathy therapeutic agent, comprising the DNA according to claim 1.

5. A method for isolating an IgA nephropathy gene comprising the DNA according to claim 1 from leukocytes obtained from a patient with IgA nephropathy, comprising conducting a differential display method.

6. An in vitro method for detecting mRNA of an IgA nephropathy-related gene, comprising using the DNA according to claim 1.

7. DNA according to claim 1 for use in a method for inhibiting transcription of an IgA nephropathy-related gene or translation of mRNA of an IgA nephropathy-related gene.

8. Use of the DNA of claim 1 or 2 for the preparation of a medicament for the treatment of IgA related nephropathy.

## Patentansprüche

1. DNA, die mit dem IgA-Nephropathie-Gen verwandt ist, umfassend die Nucleotidsequenz wie dargestellt in SEQ ID NO: 31.

2. Rekombinante DNA, umfassend die DNA nach Anspruch 1 und einen Vektor.

3. Diagnostisches Mittel für IgA-Nephropathie, umfassend die DNA nach Anspruch 1.

4. Therapeutisches Mittel für IgA-Nephropathie, umfassend die DNA nach Anspruch 1.

5. Verfahren zum Isolieren eines IgA-Nephropathie-Gens, umfassend die DNA nach Anspruch 1, aus Leukocyten, die von einem Patienten mit IgA-Nephropathie erhalten wurden, umfassend das Ausführen eines Differential Display-Verfahrens.

6. In vitro-Verfahren zum Nachweis von mRNA eines IgA-Nephropathie-verwandten Gens, umfassend die Verwendung der DNA nach Anspruch 1.

7. DNA nach Anspruch 1 zur Verwendung in einem Verfahren zur Hemmung der Transkription eines IgA-Nephropathie-verwandten Gens oder der Translation der mRNA eines IgA-Nephropathie-verwandten Gens.

8. Verwendung der DNA nach Anspruch 1 oder 2 für die Herstellung eines Arzneimittels zur Behandlung von IgA-verwandter Nephropathie.

## Revendications

1. ADN lié au gène de néphropathie à dépôts mésangiaux d'IgA comprenant la séquence nucléotidique représentée par SEQ ID NO : 31.

2. ADN recombinant comprenant l'ADN selon la revendication 1 et un vecteur.

3. Agent de diagnostic de la néphropathie à dépôts mésangiaux d'IgA comprenant l'ADN selon la revendication 1.

4. Agent thérapeutique de la néphropathie à dépôts mésangiaux d'IgA comprenant l'ADN selon la revendication 1.

5. Procédé pour isoler un gène de néphropathie à dépôts mésangiaux d'IgA comprenant l'ADN selon la revendication 1 issu de leucocytes obtenus sur un patient ayant une néphropathie à dépôts mésangiaux d'IgA, comprenant la mise en oeuvre d'un procédé de présentation différentielle.

6. Procédé in vitro pour détecter un ARNm d'un gène lié à la néphropathie à dépôts mésangiaux d'IgA comprenant l'utilisation de l'ADN selon la revendication 1.

7. ADN selon la revendication 1 destiné à être utilisé dans un procédé pour inhiber la transcription d'un gène lié à la néphropathie à dépôts mésangiaux d'IgA ou la traduction d'ARNm d'un gène lié à la néphropathie à dépôts mésangiaux d'IgA.

8. Utilisation de l'ADN selon la revendication 1 ou 2 pour la préparation d'un médicament pour le traitement de la néphropathie liée à des dépôts mésangiaux d'IgA.


### SEQUENCE LISTING

(1) GENERAL INFORMATION:
   (i) APPLICANT:
      (A) NAME: KYOWA HAKKO KOGYO CO., LTD.
      (B) STREET: 6-1, Ohtemachi 1-chome, Chiyoda-ku
      (C) CITY: Tokyo
      (E) COUNTRY: JP
      (F) POSTAL CODE (ZIP): 100
   (ii) TITLE OF INVENTION: IgA Nephropathy-related genes
   (iii) NUMBER OF SEQUENCES: 94
   (iv) COMPUTER READABLE FORM:
      (A) MEDIUM TYPE: Floppy disk
      (B) COMPUTER: IBM PC compatible
      (C) OPERATING SYSTEM: PC-DOS-/MS-DOS
      (D) SOFTWARE: PatentIn Release #1.0, Version #1.30 (EPO)
   (v) CURRENT APPLICATION DATA:
      APPLICATION NUMBER: EP 97 94 6124.1
   (vi) PRIOR APPLICATION DATA:
      (A) APPLICATION NUMBER: JP 8/325763
      (B) FILING DATE: 05-DEC-1996
   (vi) PRIOR APPLICATION DATA:
      (A) APPLICATION NUMBER: PCT/JP97/04468
      (B) FILING DATE: 05-DEC-1997
         2) INFORMATION FOR SEQ ID NO: 1:

   (i) SEQUENCE CHARACTERISTICS:
      (A) LENGTH: 2689 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (ix) FEATURE:
      (A) NAME/KEY: CDS
      (B) LOCATION:107..535
   (xi) SEQUENCE DESCRIPTION: SEQ ID NO: 1:
   GTTGGAGGTT CTGGGGCGCA GAACCGCTAC TGCTGCTTCG GTCTCTCCTT GGGAAAAAAT     60
   AAAATTTGAA CCTTTTGGAG CTGTGTGCTA AATCTTCAGT GGGACAATGG GTTCAGACAA    120
   AAGAGTGAGT AGAACAGAGC GTAGTGGAAG ATACGGTTCC ATCATAGACA GGGATGACCG    180
   TGATGAGCGT GAATCCCGAA GCAGGCGGAG GGACTCAGAT TACAAAAGAT CTAGTGATGA    240
   TCGGAGGGGT GATAGATATG ATGACTACCG AGACTATGAC AGTCCAGAGA GAGAGCGTGA    300
   AAGAAGGAAC AGTGACCGAT CCGAAGATGG CTACCATTCA GATGGTGACT ATGGTGAGCA 360
   CGACTATAGG CATGACATCA GTGACGAGAG GGAGAGCAAG ACCATCATGC TGCGCGGCCT 420
   TCCCATCACC ATCACAGAGA GCGATATTCG AGAAATGATG GAGTCCTTCG AAGGCCCTCA 480
   GCCTGCGGAT GTGAGGCTGA TGAAGAGGAA AACAGGTGAG AGCTTGCTTA GTTCCTGATA 540
   TTATTGTTCT CTTCCCCATT CCCACCTCAG TCCCTAAAGA ACATCCTGAT TCCCCCAGTC 600
   TTCAAGCACA TGAATTCAGA ATGAAAGGTT TGCCATGGCT AAGGAATGTG ACTCTTTGAA 660
   AACCATGTTA GCATCTGAGG AACTTTTTTA AACTTTGTTT TAGGGACTTT TTTTTCCTTA 720
   GGTAAGTAAT GATTTATAAA CTCCTTTTTT TTTTTTTGAC TATAGTCGGT TGCATGGTTA 780
   CTTTAAGCGT GGAATCAAAT GGAGTGGCAT TTAGTTCAGG CGGCTTGTTC CTTGCCATGG 840
   CAAAGTATCA AGAAGATCCC CAAGTCAAGT CACATTTGTA AAGCTGCTTC CCAATTGGCT 900
   TTGTCACGCA GTGTTGAAGC AGTGGGAGAG AGATTCACCT GTTATAAAGG AACTGACTAA 960
   CACAAGTATC CCGTCTATAT CTGAATGCTG TCTCTAGGTG TAAGCCGTGG TTTCGCCTTC 1020
   GTGGAGTTTT ATCACTTGCA AGATGCTACC AGCTGGATGG AAGCCAATCA GGTTGCTTCA 1080
   CTCACCAAGT CTAGATATTC ATGAAAATGG AACAAGTCTG TACAATTTTA AAAAAAGGTT 1140
   GAAGGAGTGG TTTGTTCCAA AGGAGTGACT TTTTTTTAAA AAAAAAAGCT TTGTATATAT 1200
   TAAAATTGAT GTTACTAGAA TAAGTACAGT ACCAAGGACT TCATTATAGA ATTTGTTCTG 1260
   CCTTTAAACA TGGCTACCTA CCTGGCAGGG CTTTGTTAAC TACTGAATAC CTGTCTGGTA 1320
   ATCACTAAAA CATCTTAATG TTTCCCTTTT TTCTAGTTTG TTATATTCCT ATTATGTCCA 1380
   TTGAGAGTAA GCTTAGTATA TCAAACTCTC CATTTGACAG TGAAGAGAAC ATAGTGAAAG 1440
   TCTGTGGCGG CATTTTTATA AGTAATTCCT TATTTCTGCC TGAAGACCAC AAAGCCTCCT 1500
   GGAGGCGTAA CTGCTCAGAC CGGTCTTCAG GGAATATTTA AGGACTTAGT GGAATTTATG 1560
   AACAATAAGT CTGATGAGAT TAGCCTGGGA GTGGTGTCCT GCAGCTGTCT AATCTAGTTA 1620
   GAGTGGCATT AACATTCTAA TCTCCTTGAG AATGCCTTTT ATAGTCTGTT CAAAGCAAGT 1680
   CATTGATGGT TCTTCGAGGT AGTGTTAACT GAAGTGTTCT TCAGTTTGTC AAGATAATGT 1740
   TCAGTGCTTG GCACTTAAAT AACATTTTTT GCAAGAACTC CAAGGCACAT TATTGAATGC 1800
   CTTTAACCAA GTGCATTCTG GGAAGTTTGC TTGACTCATT ATCTTGCTTT TCTGCAGCAT 1860
   TCTGTGATTT GAGTCATCCA TGAATCCATG AATAAAAGTT ACATTCTTTG ATTGGTAATA 1920
   TTGCCATTTA TAACAAGACT CACTAATGAG GGTATCACTT TGACTGACTG ATTTGTTAAA 1980
   GTTTTTAAGC CTCTCATTTT CCTAACCCAG AAATCACAGC CTGATTTTAT TAAAAGTAGA 2040
   GCTTCATTCA TTTCATACCA TAGATACCAT CCTAGTAAAT CCAGAACATA TACAAGGTTC 2100
   ATGTGAGTCT GCTTTCTTGA CATGATAGCA TTGTTTGATG CAGTGGATAT GTCAGAATGA 2160
   CTAACCTAGG AGTTTAAAAC TCCTAAGAAA CTAAAACCTG TAAGACATTT AAAAGTCTCC 2220
   ACAATTTTAA TGTATACAAA GCTATGTTAC TGTGTAACAC ATTACAGTTC AAATTCACTC 2280
   CAGAAATAAA AGGCCAGTAG GATTAGGGAC TCACTGGTAG TTTGGAGTCT CCCAGCACAC 2340
   ATCCCTCCTA GTGGGATGAT CTATTCACAT ATCTCCCAGC TTTTTTATTT TTGCTTCTGT 2400
   ATATCACAGT GAGTGGATGG CCCTTCAGCT TTTTCTCTCC TGGCCAGACA TGCAGTCTTG 2460
   CCTTTAGATA TCGCAGAGAC AAAATTCACA GCATGTCTTA AATCTTCCAG GATTTGCAAG 2520
   AACCAAATTG CTCAACAGTA TGTATGTTTA GAGGGGTTAG ACTCCTTTTT AAAATCTGGA 2580
   TATCTAACCA CCTACTTAAA TCTGTTTGAT AGTGTCAAAC CACCCCCACC CTTGATCCTC 2640
   CCACCCCCAA AAAAAAAAAA AAAAAAAAAA AAAAAAAAAA AAAAAAAAA 2689
(2) INFORMATION FOR SEQ ID NO: 2:
   (i) SEQUENCE CHARACTERISTICS:
      (A) LENGTH: 155 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 2:
   CACTTATAAA ATGTTAGGGC TTAATATTAT TCATAGATCG AGGATAGTTT CATTCTTAGT 60
   CGCCTCCTTA GTCACTCTTC CTATACCAAT CTGAGACCAT TTTACAATTT AGAAAAGACA 120
   AATAACTGGT TGGGTTACTT GATAGTATAA TAACC 155
(2) INFORMATION FOR SEQ ID NO: 3:
   (i) SEQUENCE CHARACTERISTICS:
      (A) LENGTH: 305 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 3:
   TCATGAAGTG AAGCCAACTG TTTAGACTAG AATGTTATGA GATTAAACCC ACNNNNNNTT    60
   ATTCATAGAC ATAAACCCTC ATTTTAATTA GTGGATCTGG ATTTTTGTCA TATGTGGAAT   120
   CATAATTTAA ACAAAATCAA CTAAGATGAT CCAAGTTCCA CACAACTGCA CTTCAATATT   180
   CAAGTCGGTG TGAAGATGCC TGACTACTGC GTCACAAGAT TCTGAGCTGT CGTAAAAAGC   240
   CTGGCTCGTG GTTTCTATTT ATAGTGTACA CATGTTGGGT TATAATCACA AACCTGGAAC   300
   TCTGT                                                               305
(2) INFORMATION FOR SEQ ID NO: 4:
   (i) SEQUENCE CHARACTERISTICS:
      (A) LENGTH: 278 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 4:
   GAAGGAGAAT ATGAAGAGGT TAGAAAAGNT CNGGNTTCTG TTGGTGAAAT GAAGGATGAA    60
   GGGGAAGAGA CATTAAATTA TCCTGATACT ACCATTGACT TGTCTCACCT TCAACCCCAA   120
   AGGTCCATCC AGAAATTGGC TTCAAAAGAG GAATCTTCTA ATTCTAGTGA CAGTAAATCA   180
   CAGAGCCGGA GACATTTGTC AGCCAAGGAA AGAAGGGAAA TGAAAAAGAA AAAACTTCCA   240
   AGTGACTCAG GAGATTTAGA AGCGTTAGAG GGAAAGGA                           278
(2) INFORMATION FOR SEQ ID NO: 5:
   (i) SEQUENCE CHARACTERISTICS:
      (A) LENGTH: 135 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 5:
   TTCTGACAAT GAGTAAGAAG AAAGAGGGTC TTGCCCTTTG GTTATTAAGA TTTATCATAG 60
   AGCAATAATA ASTAAATCGG TGTTATACCA GCACAGAGAT TAGACAAATA AACCAAGGGA 120
   CTGGACTAAA TAAGC 135
(2) INFORMATION FOR SEQ ID NO: 6:
   (i) SEQUENCE CHARACTERISTICS:
      (A) LENGTH: 197 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 6:
   ATGGTACCCA GTTTCAAATT AACATGGTTA TTTTACTTGT GTTCCCAAAT TTAACATTAG 60
   GGAATTTTTG GTTGTGGGTC TGTTATCACT AGAAAAATAT ATATATTGGT GCTGAAGATA 120
   ATTTTGAGAT AATTAGACAA GACAGTTTAG CATTTACAAG AACAAGTTTG GCAGTTGAAG 180
   AATCTATTTA TATGACT 197
(2) INFORMATION FOR SEQ ID NO: 7:
   (i) SEQUENCE CHARACTERISTICS:
      (A) LENGTH: 137 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 7:
   CCACCGCACC TGGCTGATGC TTTTCTATCT GACTTCTTTC AGAGGACCCT GAAAGACACT 60
   AAGTGGAATC TTTCCTTGAA GTCTTCCAAG CTAAAACAAT TCTCTGGAAA GATCACCTCT 120
   GTTCAGTCCT GGTCTCT 137
(2) INFORMATION FOR SEQ ID NO: 8:
   (i) SEQUENCE CHARACTERISTICS:
      (A) LENGTH: 274 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 8:
   CGTTTACAGA TTCTCTTGCG GCTGGCGGTG GAACTACAAA GGGATCGGTG CCTATATCAC 60
   AATACCAAAC TTGATAATAA TCTAGATTCT GTGTYTCTGC TTATAGACCA TGTTTGTAGT 120
   AGGTAAGAGG AAAACTTCCT ATATTCTGAA ACAGCCTAAC ATTTTACAAA ATTTTAGTTT 180
   TCTTTTTTAG AGTCTTATCC TGTAGCTATA TAACAGTTCA TGTCTGATTT AGCATTTGTT 240
   CACGAGTAAA GCTGGAACTA TGAAAATTGA AAAT 274
(2) INFORMATION FOR SEQ ID NO: 9:
   (i) SEQUENCE CHARACTERISTICS:
      (A) LENGTH: 171 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 9:
   GATTAGGTGA CCTTCCTTGA ARAGCCACGG GTTTCCCATA TCGAAATGCT ATTCATTACC 60
   CGAGTCACCT ANGTTCTTAC AAAGGAAGCG AGAAAATTGC TTTTGTTGGG CCATGCCCCT 120
   TTTGCANAGG TTCCTAAGTA TAGTCGCCAN AATTTTTTTA ATGGCCTAAA G 171
(2) INFORMATION FOR SEQ ID NO: 10:
   (i) SEQUENCE CHARACTERISTICS:
      (A) LENGTH: 161 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 10:
   AGGGGCGCTT GTTCTGCTCT CAGCAGATTG GTTACACGCG TCAGGTGGTG GCGATGACTT    60
   AATTCCTAGC CCAAGAAGAA TATAATGTTA AAACTGGTTA TGTAATTTTT GTGCCTCTCC   120
   TTTTTAATGC AGTATTTAGT TCAGATGTTG GCGATTTTTC A                        161
(2) INFORMATION FOR SEQ ID NO: 11:
   (i) SEQUENCE CHARACTERISTICS:
      (A) LENGTH: 323 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 11:
   TATAAGGWGG GAACCTTACT ATCTCTAATG ACCTTACTGA TGCTGACTTT AATACTCTGT    60
   GAAGGTTAGA GTTCAGTGAA TGTTACCTAG AAACAGCCCC GGCTGTGGAA TACTTTATTC   120
   TTAGCCCTAT ATTTGGGGTT TGGATGTCCA CTGTGCTGGT TCCCAGAGAT AGTAAGGGGA   180
   TGAGAGTATT GGTTACATCT CCTGACCCAC ATACTTAAGA TCCAGATGAA CAAGACAGTT   240
   TTCACTCCTG CTTGGTAGAA CCTATTTGYK SHAGGAAACA GYTCCTAAAG AATGGTTCTA   300
   GCCAGACCCT GTCGYTACCA GAA                                            323
(2) INFORMATION FOR SEQ ID NO: 12:
   (i) SEQUENCE CHARACTERISTICS:
      (A) LENGTH: 138 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 12:
   AGTATGACAA ATAGTTTCTG CCTGATTGGT GAGATTTGGG ATGGGCCCCC ACTTTGTTTC    60
   TCTTTCTGCA TAAAAATTTC AACATTTTTA CAAAATTTTC AAAAACTTCT CCTCAGTCTG   120
   TACATCTTTG TTAATCAG                                                 138
(2) INFORMATION FOR SEQ ID NO: 13:
   (i) SEQUENCE CHARACTERISTICS:
      (A) LENGTH: 244 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 13:
   TACTCTTCAA CCATGATTTT TCTCTGATGG CCTGTGTGAA CAGATTAATG GTGTCCATCT    60
   AATTCCTTCC CCACTGGGGG AAAGCAAATC ATCAGGCCCA TTGCAAAAAC TGCTCTTGGT   120
   TGAGCTTCCT GCCTTAAATC ATACCCACAG TGAATGGCGT CCCTTTATCA CCGCTAATGA   180
   CTCTGACATC TCTCTCCACT CACATGTGAG CCTCCTCAGC TCTCGANAAA CAAGTCNGTC   240
   TCGG                                                                244
(2) INFORMATION FOR SEQ ID NO: 14:
   (i) SEQUENCE CHARACTERISTICS:
      (A) LENGTH: 135 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 14:
   TGATCCCCAC AATTTCTTGT GATTGGTGAG GAACTATAAA TGACTCCCAT CCAAGCTTAT    60
   ACCAGAAAAA AGGAGCACAT TTTCTACAAA TTATATCATT TTTAATCCAT TACCACATTA   120
   TTTTAGGGGA ACTAC                                                    135
(2) INFORMATION FOR SEQ ID NO: 15:
   (i) SEQUENCE CHARACTERISTICS:
      (A) LENGTH: 258 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 15:
   TCTCAGAAAA CTCCAGATCA AATGAGATGA GTATGGTGNN NAGGGCTGGC AATTAGAGGA    60
   TACTCTCCAA TGGTGATGAA GGGAGATGTC TGGGGGAAAT CCAGCAGGAT GTTGATTTAG   120
   TATGTACACA GTGAGAGGAT ACTTGTAGAG AACCTAGAAT CTTCTCTGAA TGTGACGGGC   180
   CCTCAGAGAT AATTGTTAAC AGATAAGTGG ATGATTAAAT ACACTTCCTC CAGTAGGCTA   240
   GATGTTAAGA CGGAGATC                                                 258
(2) INFORMATION FOR SEQ ID NO: 16:
   (i) SEQUENCE CHARACTERISTICS:
      (A) LENGTH: 219 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 16:
   CTGAGAGGAG CCATGTATAC AAACCACTTT TTCTAACATG GTCTTTATTA AACTTTGAAT      60
   ATAAGTACAC CTGCTCGAAG TGTTCATCTA TATTATTTAA GAACAAGCAA CTGTAAAACA     120
   GTAAAATCAC AAAAGGTAAG TTGTTGGAAG ACAACAAAAA AGAATTACTA TATCTGATCC     180
   TGCGTGTTTA TTTTAGAATC TGTTAATAGG CCTACAGCT                            219
(2) INFORMATION FOR SEQ ID NO: 17:
   (i) SEQUENCE CHARACTERISTICS:
      (A) LENGTH: 191 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 17:
   ACAGTGAGTG TGGCTGAAAC CTAAGCTGAA GGAAGGGAGG AGCAGGCACT GCCATGAGGG      60
   GTCCCTGGAC AGAAACTCTT CAGCAGGCCT TGAAGTTTAG TTCAGGGGCT ACATGGAATA     120
   CCACTATTTA GCACACAGGT GTGATCTGAG GTGAGGGACT ACCTTTTCGA TCTTGGTTTT     180
   CTCATTTATT T                                                          191
(2) INFORMATION FOR SEQ ID NO: 18:
   (i) SEQUENCE CHARACTERISTICS:
      (A) LENGTH: 148 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 18:
   CTGGAGGTGA AGGGAAGGAA AGAAAGGAAA AACTATCTAC CTGGCAGGAA AAGAGATAAG 60
   CTCCCAAGAA CACCAAAGCA GATGATGAGT CTAGCTCTAC CCAGCCTTCC TCCCCACGAA 120
   TCCAGATCAT AGTAAGAAAC TCTGGGCT 148
(2) INFORMATION FOR SEQ ID NO: 19:
   (i) SEQUENCE CHARACTERISTICS:
      (A) LENGTH: 306 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 19:
   CCACCACCAG AAATGAACAA AAAGCATTTT ACCTAAAAAT ACACCAGCAA AATGTACTCA 60
   GCTTCAATCA CAAATACGAC TGCTTAAAAC CGCAGAAATT TCCTCAACAC TCAGCCTTTA 120
   TCACTCAGCT GGATTTTTTC CTTCAACAAT CACTACTCCA AGCATTGGGG AACACAACTT 180
   TTAATCATAC TCCAGTCGTT TCACAATGCA TTCTAATAGC AGCGGGATCA GAACAGTACT 240
   GCATTTACTT GCCAACAGAA CAGACAGACC TGAAGTCAAG ACAACTGCAT TCTCTGTGAA 300
   GTCTGT 306
(2) INFORMATION FOR SEQ ID NO: 20:
   (i) SEQUENCE CHARACTERISTICS:
      (A) LENGTH: 357 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 20:
   GTAGCATTTT GGCAGAACCA TTGTTAATTA AAGGGACTTY TGGACCGCAA CYTTAATGTA 60
   CCAGATTATT GAGCRGCCCA ATGAATGCTT CATTCTCATT GTTTAAGGTG CTGCTTTGAT 120
   TTTTTTTTCA ATTCTTTGTA CTATTTTTTA TTTTTTGGAG AGGCACATCC CCAAATTTGG 180
   ATGAGGTATT TGTTGATAAA TAATTCATCA ATTTCCACAA TGCAGACAAA AATGTCTGCC 240
   CAGAGTGGAA AAATAAAACA AGGGGGAGAA GAGTTTGAGT AACGGAGAAG TTCTGTGGAA 300
   TCCTAGTGAC AAAAGTTGAG AAACTACCTT TAAATAAGAC AGTGAGGTAA CAAATGT 357
(2) INFORMATION FOR SEQ ID NO: 21:
   (i) SEQUENCE CHARACTERISTICS:
      (A) LENGTH: 219 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 21:
   TGGAATAGCC AGGAGAATTC TGGAAAAGTA GAATAATGAG GTAGGGCTTC CCTTCGCTAT 60
   TTTGAAGTGC AGATTACACT ATGTAAAACC ATTAGGAACT GGCACGTGAA TAGACAGATC 120
   AATAGTTAAT AGCTGTATTA GCCAGAAAAT GGTGTAAGGA CAACAGGCTA ACTAACCCTG 180
   TCACTTGTTA TGCTAAAATT AAGTCTAGAT AGAGTCCTC 219
(2) INFORMATION FOR SEQ ID NO: 22:
   (i) SEQUENCE CHARACTERISTICS:
      (A) LENGTH: 251 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 22:
   TGAAAGGGGA ATAGAAGCAC AAGAGTCAGT AATCAATAAC AAACAACTCA AGGTGCTCCT     60
   TCCTTACACT GGTGTTCCCC AAAGTGAGGT GAATTGCCAG CCACTGGGAG TCAGGGCCAG    120
   TTACATAAGA CATTCTCGGT AAGCCCCCTT TGGGTATCCC AAATAAGGAC TGGGGTGGGT    180
   TTATGTGTAG TCCATTATTA ACAACTAAAC GAACAAACCT AGTGAATTGC AATAAATTCA    240
   CACCAACAGA A                                                         251
(2) INFORMATION FOR SEQ ID NO: 23:
   (i) SEQUENCE CHARACTERISTICS:
      (A) LENGTH: 233 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 23:
   GTTGAAAGAG TCCTTGGAAG GCTTTTAGAC CAAACCCCTC TGCATGCTCA ARCCTTGGGT     60
   ACAGGATTTC TAAGAAGTGG AACAGTCTCC AGGGGTGTGG ARCTCATCGC TCAAGGCAGG    120
   TTATCTTATC TGAATAATTT TGTCTGTTGA CTATTGGGAT AGTTCTCCTT CAGATGAGCT    180
   GAAATTTTCT CCATAGCTTC CTCTATTAAA CCCAATTCCA CTTCTCAGGG TCA           233
(2) INFORMATION FOR SEQ ID NO: 24:
   (i) SEQUENCE CHARACTERISTICS:
      (A) LENGTH: 176 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 24:
   CAAAAGCGCT GAAGTTAAGC ATTAATACGC CAGATTCATG ATTTATGATC AGTATCCAAA 60
   ACTCCAACTA CAAACAATGC AAAGTAGTGC TCCTCAGTAT TATTTTTGCA ATTGTTAGTA 120
   ATGTTAAGCA TCAAGGAAAA TAAAACACAT CATTGCACAT TACAGCCGCA AAAAAC 176
(2) INFORMATION FOR SEQ ID NO: 25:
   (i) SEQUENCE CHARACTERISTICS:
      (A) LENGTH: 241 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 25:
   AGAGAGTAAA GCAAGCTATT TTGACAGCAA CCTAATAACA GCTGTCTTCT TCCACTTCTT 60
   GGCTAACTCA TCCCCCAGAT AGCCTTCTTT TCTCTTATCA ATTCCCTGTT GCAACAATAA 120
   TAAATGCCAC ACCTGATGGA GTCATTAGGC ACTTTCCTAG TGACAAGTGC CTAGGACAGA 180
   GGAGAAAACA AAGAAACACT GACAACCACT GAAAACTGAC ATATCAGGCC AGGCATGTCA 240
   C 241
(2) INFORMATION FOR SEQ ID NO: 26:
   (i) SEQUENCE CHARACTERISTICS:
      (A) LENGTH: 217 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 26:
   GCTGGAGAGG TGGTGATGTT GCTGAATAAT TGCTTTTTAA AGCTGGAGGG GACTTCCAAG 60
   AGTCTCTCAT TTAAGAARAA AAATTAAAGA CATAATTGGT AACGGTTTTG ACTGCTGCAG 120
   AGGCAACACT TTGCTCACAA TCCTACAGAT CTACTTCACC TGTAACTACA ATTTTCCTGA 180
   AGACATAGAA GAAAAATCAA TTGTTCTAAT CCATATG 217
(2) INFORMATION FOR SEQ ID NO: 27:
   (i) SEQUENCE CHARACTERISTICS:
      (A) LENGTH: 233 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 27:
   AATCTTAGCA TAATGCTTCC TGGGAAATTC TGAAATTGAT TCCATTTCTG CCGTTACAAA 60
   CACACACGAA GTTCCTAGTT CACTGGGACT TCCTGATTTG TTCTTTTAGC TTGCTCCTTC 120
   TCACCTAGAA GCTCTGTTTA TTTCTGAGCA ACCCTGGGGC TTGTCTCATA GGACAGGATT 180
   TATTTATCTC ATCAAGGCTG AGTGTGCCTT AGGAAGTCAT AAACATAAAA AGA 233
(2) INFORMATION FOR SEQ ID NO: 28:
   (i) SEQUENCE CHARACTERISTICS:
      (A) LENGTH: 228 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 28:
   TATAGACAGG GTAGGGACGA TTAGCCCCTC GACAACTTTT CACAAATATA CACACGTTTA 60
   ACTACCTCTC AGGTCATGAT AAAGACCGGC CGGGCAGAAA CACTGTAATC CCAGCTACTC 120
   GGGAGCCTGA GGCATGAGAA TCACTTGAAC CTGGGAGGTG GAGGTTGCCA TGAGCCGAGA 180
   TCACGCCATT GCACTACAGC CTTGGCGACA AGAGTGAAAC TCCATCTG 228
(2) INFORMATION FOR SEQ ID NO: 29:
   (i) SEQUENCE CHARACTERISTICS:
      (A) LENGTH: 298 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 29:
   GCTTATGATT ACAAACATCC CTCATATGAA AATCTCAGCA TTTNCTGGCT GCTGCCTTCA 60
   ATCGCTTTTT CTGAAATAGG TATCCCTTGA TGTCGACTAT TTGATTTCAG CCAGTCGTTT 120
   CTCTCTGGCA GTGCTCCCTG CAAATGTGTC CTTTCAAGAA AACAAAACCT GCAAGTGGCT 180
   TGTAATGTAC CATGACCTTA TCATGTGAAG GACAAATGGC TCTTGTGCTT ATTAGATAGC 240
   AGATGAACTG ATGAACTGAA TTCTTGGTCT GAAGCTTTGA TAAGGTCAGA TGTCTTTG 298
(2) INFORMATION FOR SEQ ID NO: 30:
   (i) SEQUENCE CHARACTERISTICS:
      (A) LENGTH: 291 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 30:
   ACTTCGAAGG GAAAAAGAGG AAGGAAAAGG ACTGTTAATA AAATAACAAA GGCAGCAATC    60
   AGAATGAACC AGAGCCAGGA CAGCGTAAAG GCTAGGTTCA CAGTGAGATG AAAGAACCTG   120
   AAAACAAGTT TAAAACTCAA AAGAGGATTA TTCTCAAGTT ATACTACAGT GAAAAAACAT   180
   GGAAAAACAC AAAAAGGACA GGCAATAAGG CACAGGCATA CATACAAGGC AAATTGTAAC   240
   ACAATATTTA CTTGCAAAAG AGCCCACAGA GACATGTCAA TGAAGTCATA G            291
(2) INFORMATION FOR SEQ ID NO: 31:
   (i) SEQUENCE CHARACTERISTICS:
      (A) LENGTH: 869 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Human
      (G) CELL TYPE: Leukocyte
   (xi) SEQUENCE DESCRIPTION: SEQ ID NO: 31:
   CGCGTCCGGT GCCTGGCTGC AGTAGCAGCG GCATCTCCCT TGCACAGTTC TCCTCCTCGG    60
   CCTGCCCAAG AGTCCACCAG GCCATGGACG CAGTGGCTGT GTATCATGGC AAAATCAGCA   120
   GGGAAACCGG CGAGAAGCTC CTGCTTGCCA CTGGGCTGGA TGGCAGCTAT TTGCTGAGGG   180
   ACAGCGAGAG CGTGCCAGGC GTGTACTGCC TATGTGTGCT GTATCACGGT TACATTTATA   240
   CATACCGAGT GTCCCAGACA GAAACAGGTT CTTGGAGTGC TGAGACAGCA CCTGGGGTAC   300
   ATAAAAGATA TTTCCGGAAA ATAAAAAATC TCATTTCAGC ATTTCAGAAG CCAGATCAAG   360
   GCATTGTAAT ACCTCTGCAG TATCCAGTTG AGAAGAAGTC CTCAGCTAGA AGTACACAAG   420
   GTACTACAGG GATAAGAGAA GATCCTGATG TCTGCCTGAA AGCCCCATGA AGAAAAATAA   480
   AACACCTTGT ACTTTATTTT CTATAATTTA AATATATGCT AAGTCTTATA TATTGTAGAT   540
   AATACAGTTC GGTGAGCTAC AAATGCATTT CTAAAGCCAT TGTAGTCCTG TAATGGAAGC   600
   ATCTAGCATG TCGTCAAAGC TGAAATGGAC TTTTGTACAT AGTGAGGAGC TTTGAAACGA   660
   GGATTGGGAA AAAGTAATTC CGTAGGTTAT TTTCAGTTAT TATATTTACA AATGGGAAAC   720
   AAAAGGATAA TGAATACTTT ATAAAGGAWT AATGTCAATT CTTGCCAAAT ATAAATAAAA   780
   ATAATCCTCA GTTTTTGTGA AAAGCTCCAT TTTTAGTGAA ATATATTTTA TAGCTACTAA   840
   TTTTAAAATG TCTGCTGATG TATGTGGAA                                     869
(2) INFORMATION FOR SEQ ID NO: 32:
   (i) SEQUENCE CHARACTERISTICS:
      (A) LENGTH: 143 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: Human
      (G) CELL TYPE: Leukocyte
   (xi) SEQUENCE DESCRIPTION: SEQ ID NO: 32:
   Met Gly Ser Asp Lys Arg Val Ser Arg Thr Glu Arg Ser Gly Arg Tyr
   1               5                   10                  15
   Gly Ser Ile Ile Asp Arg Asp Asp Arg Asp Glu Arg Glu Ser Arg Ser
               20                  25                  30
   Arg Arg Arg Asp Ser Asp Tyr Lys Arg Ser Ser Asp Asp Arg Arg Gly
           35                  40                  45
   Asp Arg Tyr Asp Asp Tyr Arg Asp Tyr Asp Ser Pro Glu Arg Glu Arg
       50                  55                  60
   Glu Arg Arg Asn Ser Asp Arg Ser Glu Asp Gly Tyr His Ser Asp Gly
   65                  70                  75                  80
   Asp Tyr Gly Glu His Asp Tyr Arg His Asp Ile Ser Asp Glu Arg Glu
                   85                  90                  95
   Ser Lys Thr Ile Met Leu Arg Gly Leu Pro Ile Thr Ile Thr Glu Ser
               100                 105                 110
   Asp Ile Arg Glu Met Met Glu Ser Phe Glu Gly Pro Gln Pro Ala Asp
           115                 120                 125
   Val Arg Leu Met Lys Arg Lys Thr Gly Glu Ser Leu Leu Ser Ser
       130                 135                 140
(2) INFORMATION FOR SEQ ID NO: 33:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 33:
      GGGCTTAATA TTATTCATAG ATCGAG 26
(2) INFORMATION FOR SEQ ID NO: 34:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 34: GTTATTATAC TATCAAGTAA CCCAAC 26
(2) INFORMATION FOR SEQ ID NO: 35:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 35:
      GTGGATCTGG ATTTTTGTCA TATGT 25
(2) INFORMATION FOR SEQ ID NO: 36:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 36:
   GTTTGTGATT ATAACCCAAC ATGTG 25
(2) INFORMATION FOR SEQ ID NO: 37:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 37:
   GAAGGGGAAG AGACATTAAA TTATC 25
(2) INFORMATION FOR SEQ ID NO: 38:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 38:
   GCTTCTAAAT CTCCTGAGTC ACTT 24
(2) INFORMATION FOR SEQ ID NO: 39:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 39:
   GACAATGAGT AAGAAGAAAG AGGG 24
(2) INFORMATION FOR SEQ ID NO: 40:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 40:
      GTCCAGTCCC TTGGTTTATT TGTC 24
(2) INFORMATION FOR SEQ ID NO: 41:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 41:
      GGTACCCAGT TTCAAATTAA CATGG 25
(2) INFORMATION FOR SEQ ID NO: 42:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 42:
      GATTCTTCAA CTGCCAAACT TGTTC 25
(2) INFORMATION FOR SEQ ID NO: 43:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 43:
      GCTGATGCTT TTCTATCTGA CTTC 24
(2) INFORMATION FOR SEQ ID NO: 44:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 44:
      GACCAGGACT GAACAGAGGT GA 22
(2) INFORMATION FOR SEQ ID NO: 45:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 45:
      GCTTATAGAC CATGTTTGTA GTAGG 25
(2) INFORMATION FOR SEQ ID NO: 46:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 46:
      GTGAACAAAT GCTAAATCAG ACATG 25
(2) INFORMATION FOR SEQ ID NO: 47:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 47:
      GCCACGGGTT TCCCATATCG AA 22
(2) INFORMATION FOR SEQ ID NO: 48:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 48:
      GACTATACTT AGGAACCTCT GCAA 24
(2) INFORMATION FOR SEQ ID NO: 49:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 49:
      GTTCTGCTCT CAGCAGATTG GTTA 24
(2) INFORMATION FOR SEQ ID NO: 50:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 50:
      GCCAACATCT GAACTAAATA CTGC 24
(2) INFORMATION FOR SEQ ID NO: 51:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 51:
      GTTCAGTGAA TGTTACCTAG AAACA 25
(2) INFORMATION FOR SEQ ID NO: 52:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID N0: 52:
      GGAGTGAAAA CTGTCTTGTT CATC 24
(2) INFORMATION FOR SEQ ID NO: 53:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 53:
      GTATGACAAA TAGTTTCTGC CTGAT 25
(2) INFORMATION FOR SEQ ID NO: 54:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 54:
      GATTAACAAA GATGTACAGA CTGAG 25
(2) INFORMATION FOR SEQ ID NO: 55:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 55:
      GAGACAGCAT TCAGATATAG ACGG 24
(2) INFORMATION FOR SEQ ID NO: 56:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 56:
      GCGTGGAATC AAATGGAGTG GC 22
(2) INFORMATION FOR SEQ ID NO: 57:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 57:
   GATGGCCTGT GTGAACAGAT TAAT 24
(2) INFORMATION FOR SEQ ID NO: 58:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 58:
   GAGAGAGATG TCAGAGTCAT TAGC 24
(2) INFORMATION FOR SEQ ID NO: 59:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 59:
   GATCCCCACA ATTTCTTGTG ATTG 24
(2) INFORMATION FOR SEQ ID NO: 60:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 60:
   GTTCCCCTAA AATAATGTGG TAATG 25
(2) INFORMATION FOR SEQ ID NO: 61:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 61:
   GAGGATACTC TCCAATGGTG ATG 23
(2) INFORMATION FOR SEQ ID NO: 62:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 62:
   GTCTTAACAT CTAGCCTACT GGAG 24
(2) INFORMATION FOR SEQ ID NO: 63:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 63:
   GAGAGGAGCC ATGTATACAA ACCA 24
(2) INFORMATION FOR SEQ ID NO: 64:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 64:
   GCACGCAGGA TCAGATATAG TAATTC 26
(2) INFORMATION FOR SEQ ID NO: 65:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 65:
   GCTGAAACCT AAGCTGAAGG AAGG 24
(2) INFORMATION FOR SEQ ID NO: 66:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 66:
   GTCCCTCACC TCAGATCACA CC 22
(2) INFORMATION FOR SEQ ID NO: 67:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 67:
   GCTATCTACC TGGCAGGAAA AGAG 24
(2) INFORMATION FOR SEQ ID NO: 68:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 68:
   GAGTTTCTTA CTATGATCTG GATTC 25
(2) INFORMATION FOR SEQ ID NO: 69:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 69:
   GCAAAATGTA CTCAGCTTCA ATCAC 25
(2) INFORMATION FOR SEQ ID NO: 70:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 70:
   GTAAATGCAG TACTGTTCTG ATCC 24
(2) INFORMATION FOR SEQ ID NO: 71:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 71:
   GAATGCTTCA TTCTCATTGT TTAAGG 26
(2) INFORMATION FOR SEQ ID NO: 72:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 72:
   GTCACTAGGA TTCCACAGAA CTTC 24
(2) INFORMATION FOR SEQ ID NO: 73:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 73:
   GAGGTAGGGC TTCCCTTCGC TA 22
(2) INFORMATION FOR SEQ ID NO: 74:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 74:
   GCATAACAAG TGACAGGGTT AGTTA 25
(2) INFORMATION FOR SEQ ID NO: 75:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 75:
   GGTGCTCCTT CCTTACACTG GT 22
(2) INFORMATION FOR SEQ ID NO: 76:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 76:
   GACTACACAT AAACCCACCC CAG 23
(2) INFORMATION FOR SEQ ID NO: 77:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 77:
   GGGTACAGGA TTTCTAAGAA GTGG 24
(2) INFORMATION FOR SEQ ID NO: 78:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 78:
   GGAGAAAATT TCAGCTCATC TGAAG 25
(2) INFORMATION FOR SEQ ID NO: 79:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 79:
   GCTGAAGTTA AGCATTAATA CGCC 24
(2) INFORMATION FOR SEQ ID NO: 80:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 80:
   GCGGCTGTAA TGTGCAATGA TGT 23
(2) INFORMATION FOR SEQ ID NO: 81:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 81:
   GACAGCAACC TAATAACAGC TGTC 24
(2) INFORMATION FOR SEQ ID NO: 82:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 82:
   GTCCTAGGCA CTTGTCACTA GG 22
(2) INFORMATION FOR SEQ ID NO: 83:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 83:
   GAGGGGACTT CCAAGAGTCT CT 22
(2) INFORMATION FOR SEQ ID NO: 84:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 84:
   GTCTTCAGGA AAATTGTAGT TACAG 25
(2) INFORMATION FOR SEQ ID NO: 85:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 85:
   GTTACAAACA CACACGAAGT TCCT 24
(2) INFORMATION FOR SEQ ID NO: 86:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 86:
   GACTTCCTAA GGCACACTCA GC 22
(2) INFORMATION FOR SEQ ID NO: 87:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 87:
   GTTTAACTAC CTCTCAGGTC ATGA 24
(2) INFORMATION FOR SEQ ID NO: 88:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 88:
   GTCGCCAAGG CTGTAGTGCA AT 22
(2) INFORMATION FOR SEQ ID NO: 89:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 89:
   GAAATAGGTA TCCCTTGATG TCGA 24
(2) INFORMATION FOR SEQ ID NO: 90:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 90:
   GACCAAGAAT TCAGTTCATC AGTT 24
(2) INFORMATION FOR SEQ ID NO: 91:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 91:
   GAATGAACCA GAGCCAGGAC AG 22
(2) INFORMATION FOR SEQ ID NO: 92:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 92:
   GCCTTGTATG TATGCCTGTG CC 22
(2) INFORMATION FOR SEQ ID NO: 93:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 93:
   AAGAGTCCAC CAGGCCATGG A 21
(2) INFORMATION FOR SEQ ID NO: 94:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 94:
   TACCTTGTGT ACTTCTAGCT GAG 23